Europäisches Patentamt

(19) European Patent Office

Office européen des brevets

(11) Publication number: **0 117 351**
**B1**

(12) **EUROPEAN PATENT SPECIFICATION**

(45) Date of publication of patent specification: **08.07.87**

(51) Int. Cl.⁴: **A 61 F 13/18, A 61 L 15/00**

(21) Application number: **83307406.5**

(22) Date of filing: **06.12.83**

(54) **Absorptive products.**

(30) Priority: **31.01.83 GB 8302621**

(43) Date of publication of application:
**05.09.84 Bulletin 84/36**

(45) Publication of the grant of the patent:
**08.07.87 Bulletin 87/28**

(84) Designated Contracting States:
**AT BE CH DE GB LI SE**

(56) References cited:
**EP-A-0 052 403**
**GB-A-2 017 509**
**US-A-3 060 936**

(73) Proprietor: **JOHNSON & JOHNSON**
**One Johnson & Johnson Plaza**
**New Brunswick, NJ 08933-7003 (US)**

(72) Inventor: **Downie, Malcolm James**
**19 Upper Sackville Street**
**Skipton North Yorkshire, BD23 2EB (GB)**

(74) Representative: **Jones, Alan John et al**
**CARPMAELS & RANSFORD 43 Bloomsbury**
**Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 117 351**

**Description**

This invention relates to absorptive products, and more particularly to absorptive products which include a water-retardant layer. Such absorptive products include surgical dressings, and adhesive plasters, sanitary napkins, incontinence pads, etc.

It is well known to incorporate in a surgical dressing a water-retardant layer. The advantage of the water-retardant layer is that strike-through of wound exudate is delayed. This is important because wound exudate which has penetrated the outer layer of a dressing provides a path for bacterial infection from outside. A delayed strike-through, therefore, means that fewer dressing changes are required and the healing wound is less susceptible to infection.

US—A—3060936 discloses a sanitary napkin having delayed strike-through, comprising an absorbent layer and a layer of liquid repellent paper, the whole structure being embossed.

Three main types of water-retardant layer have been proposed for use in surgical dressings, namely repellent crepe tissue, plastics films, and laminates of tissue and plastics films. Repellent crepe tissue has the disadvantage that it is difficult to produce a consistently repellent product. Also, the surface of the tissue is found to become wet very quickly in use, and any areas of low repellency soon lead to strike-through. Plastics films, although effective in preventing strike-through, are generally occlusive and therefore undesirable for most applications. Laminates of tissue and plastics films suffer from the same disadvantage.

We have found surprisingly that the aforementioned disadvantages of known water-retardant layers can be avoided by the use of an embossed water-retardant fibrous web, for example, embossed water-retardant paper. One material which we have found particularly suitable for this purpose is, in fact, already known for use in cigarette filters. This is an embossed paper available from Filtrona Limited, Monarch House, 8 First Avenue, Bletchley, Milton Keynes, England. The manufacture of such paper is described in GB—A—2075328.

According to the present invention, there is provided an absorptive product as recited in Claim 1. The fibrous web may be to some extent water-retardant prior to embossing. Generally, such water-retardancy will be achieved by impregnating or coating the web with a water-retardant material. For example, the fibrous web may be paper formed from 100% bleached sulphate pulp, which paper is then sized with starch.

We have found that any inherent water-retardant properties of the fibrous web are enhanced by the embossing process. Whilst we do not wish to be bound by any theory, it is believed that the embossing process causes compaction of the fibres of the web at the most raised and depressed regions of the embossments. This causes the pores of the web in these regions to be reduced in size, thereby increasing the water-retardant properties in these regions. In those areas of the web which connect the most raised and depressed regions of the embossments, the opposite phenomenon is found. That is to say, the fibres in such areas are to some degree pulled apart by the embossing process, thus allowing greater water permeability in these areas. However, it is only the most raised (and hence most water-retardant) regions of the embossed fibrous web which come into contact with wound exudate in use. It is believed that this is responsible for the great effectiveness of the surgical dressings of this invention in preventing strike-through of wound exudate.

The areas of relatively low water-retardancy give rise to a further advantage of the use of embossed fibrous webs in surgical dressings. The areas of low water-retardancy have larger pores and therefore increased water vapour permeability. Indeed, it is particularly preferred that the embossing process should be such as to produce not only microscopic separation of the fibres of the web, but also macroscopic separation of the fibres. That is to say, it is preferred that the embossed web should have visible tears or fibrillations in the areas between the most raised and depressed regions of the embossed product.

Yet another advantage of embossing the fibrous web is that the web thereby becomes more flexible, and quieter in use.

The embossing is preferably in the form of a series of substantially parallel corrugations, which will conveniently extend in the direction of travel of the web through the embossing apparatus. The number of such corrugations per cm of web may vary. For example, the web may have from 5 to 30 corrugations/cm, e.g. 10 corrugations/cm.

The surgical dressings of the invention comprise an absorbent layer adjacent the water-retardant layer, and preferably a cover at least partially enclosing the absorbent layer and the water-retardant layer.

The cover is preferably an apertured non-woven fabric such as that sold under the Trade Mark Keybak. The fabric may be of any suitable material, such as rayon, nylon or a polyester, optionally coated with a non-adherent polymer such as polyethylene or polypropylene.

The absorbent layer may be of any type, for example, conventional absorbents such as cotton fibres, rayon, wood pulp, synthetic foams or mixtures thereof. Preferably, the absorbent layer is a mixture of cotton and rayon in which preferably more than 90% is cotton.

Preferably, a layer of tissue, for example cellulose tissue, is provided within the absorbent layer. This slightly retards the flow of exudate through the absorbent layer, and causes the exudate to spread laterally as well as vertically through the dressing.

2

An absorptive product according to the present invention is now described, by way of example, with reference to the accompanying drawings, in which:—

Figure 1 is a perspective view of a sheet of embossed fibrous web for use in an absorptive product,

Figure 2 is a diagrammatic section through a simple dressing incorporating the embossed web of Figure 1,

Figure 3 is a diagrammatic section through an alternative form of dressing, and

Figure 4 is a plan view of the dressing of Figure 2, partially cut away to show the embossed web.

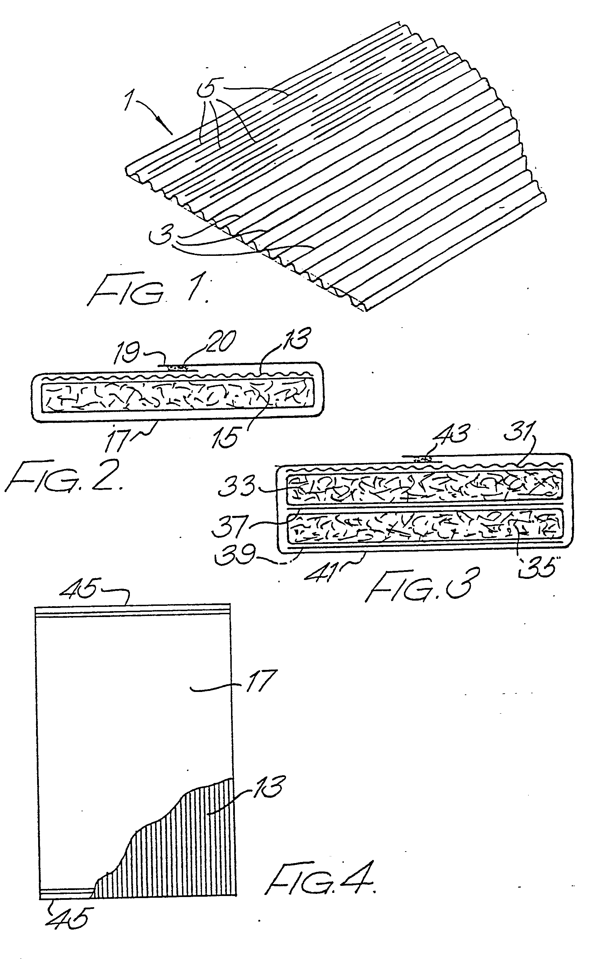

Referring to Figure 1, a paper web shown generally at 1 has embossed longitudinally extending ridges 3. The web is embossed so as to produce a large number of small longitudinally extending tears 5 in the parts between the most raised and depressed regions of the web.

The dressing shown in Figure 2 comprises a water-retardant layer 13, and an absorbent layer 15, enclosed in a cover 17. The water-retardant layer 13 is a web of embossed paper such as that illustrated in Figure 1, and the absorbent layer is a pad of cotton pulp. The cover is an apertured non-woven fabric having an overlap 19 which is secured by means of a line of hot-melt adhesive 20.

Figure 3 shows a dressing comprising a water-retardant layer 31 and two absorbent layers 33, 35. Between the absorbent layers is a layer of absorbent crepe tissue 37 and a further layer of absorbent crepe tissue 39 is placed beneath the second absorbent layer 35. The water-retardant layer, the absorbent layers, and the layers of absorbent crepe tissue are contained within the cover 41 which is secured by means of a line of hot-melt adhesive 43 as in Figure 2.

The dressings of Figures 2 and 3 may be formed continuously by conventional means, and then severed to form individual dressings. If desired, the severed ends 45 of the dressings may be closed for example by the application of a hot-melt adhesive.

The dressings may be affixed to a patient by any suitable method such as by bandaging, or by means of adhesive tape. If desired, the dressing itself may include an adhesive surface such as a pressure sensitive adhesive coating.

The delayed strike-through of the dressings in accordance with the present invention is illustrated by the following Example.

Example

A Surgipad (Registered Trade Mark) dressing (made by Johnson & Johnson) was modified by including a layer of Myria (Registered Trade Mark) paper made by Filtrona Limited. The paper had a weight of 37 $g/m^2 \pm 5$ $g/m^2$ and approximately 10 corrugations/cm. The modified dressing was compared with a conventional Surgipad dressing, and with a Surgipad dressing incorporating a layer of repellent tissue, by means of the following test. The dressings were each placed between two curved Perspex plates which were held together by elastic bands (Perspex is a Registered Trade Mark). The lower plate was provided with a central aperture in communication with a reservoir of test fluid. The test fluid was chosen to simulate a typical wound exudate, and had a specific gravity of 1.060 to 1.075, a viscosity of 5.2 to 6.1 centipoises, and a surface tension of 0.044 to 0.050 N/m. The time taken for the test fluid to strike through to the upper Perspex plate under the pressure of a 2.5 cm (1 inch) head of fluid was measured. Each of the dressings had previously been sterilised by steam at 134°C for three minutes. The results were as follows:—

| Dressing | Time to strike through (secs) |
|---|---|
| Surgipad dressing (no retardant layer) | 11 |
| Surgipad dressing, including repellent tissue layer | 30 |
| Surgipad dressing, including a layer of Myria paper | 171 |

The results show that the use in a surgical dressing of a layer of embossed water-retardant paper provides greatly delayed strike-through. The dressing was nonetheless highly permeable to water vapour.

Various types of absorptive products may be provided in accordance with the present invention including adhesive plasters, surgical sponges etc., as well as other exterior dressings.

**Claims**

1. An absorptive product having delayed fluid strike-through comprising an absorbent layer (15) and a layer (13) of water-retardant embossed sheet material overlying one major surface of said absorbent layer

## 0 117 351

(15), characterised in that said layer (13) of embossed sheet material consists of porous fibrous sheet material having outer surface areas of relatively low porosity and inner surface areas of relatively high porosity.

2. An absorptive product according to claim 1 wherein said embossed material (13) is a water retardant paper.

3. An absorptive product according to claim 2 wherein said paper (13) is sized with starch.

4. An absorptive product according to any of claims 1 to 3 wherein said embossed material (13) includes a series of substantially parallel corrugations (3).

5. An absorptive product according to claim 4 wherein said embossed material (13) has 5 to 30 corrugations (3) per centimetre.

6. An absorptive product according to any preceding claim at least partially enclosed in a fluid permeable wrapper (17).

7. An absorptive product according to claim 6 wherein said wrapper (17) is nonwoven fabric.

8. An absorptive product according to any preceding claim wherein a layer (37) of tissue is included within the absorbent layer (33, 35) between the major surfaces thereof.

9. An absorptive product according to any preceding claim including an adhesive coating on at least a portion of the outer surface (41) of said product opposite said embossed material (31).


## Patentansprüche

1. Ein absorbierendes Produkt mit verzögertem Durchschlagen von Flüssigkeit, umfassend eine absorbierende Schicht (15) und eine Schicht (13) aus wasserrückhaltendem, geprägtem, bahnartigem Material, welche letztere Schicht (13) über einer Hauptoberfläche der genannten absorbierenden Schicht (15) liegend angeordnet ist, dadurch gekennzeichnet, daß die genannte Schicht (13) aus geprägtem, bahnartigem Material aus porösem, faserhältigem, bahnartigem Material besteht, welches Material Außenseitenbereiche von relativ niedriger Porosität und Innenseitenbereiche von relativ hoher Porosität aufweist.

2. Ein absorbierendes Produkt nach Anspruch 1, worin das genannte geprägte Material (13) ein wasserrückhaltendes Papier ist.

3. Ein absorbierendes Produkt nach Anspruch 2, worin das genannte Papier (13) mit Stärke geschlichtet ist.

4. Ein absorbierendes Produkt nach einem der Ansprüche 1 bis 3, worin das genannte geprägte Material (13) eine Reihe von im wesentlichen parallelen Wellen (3) aufweist.

5. Ein absorbierendes Produkt nach Anspruch 4, worin das genannte geprägte Material (13) 5 bis 30 Wellen (3) je cm aufweist.

6. Ein absorbierendes Produkt nach einem der vorhergehenden Ansprüche, welches wenigstens teilweise von einer flüssigkeitsdurchlässigen Umhüllung (17) umschlossen ist.

7. Ein absorbierendes Produkt nach Anspruch 6, worin die genannte Umhüllung (17) ein Faservliesstoff ist.

8. Ein absorbierendes Produkt nach einem der vorhergehenden Ansprüche, worin eine Schicht (37) aus Tissuepapier innerhalb der absorbierenden Schichten (33, 35), und zwar zwischen den Hauptoberflächen der absorbierenden Schichten, eingeschlossen ist.

9. Ein absorbierendes Produkt nach einem der vorhergehenden Ansprüche, welches einen klebenden Überzug auf wenigstens einem Teil der Außenseite (41) des genannten Produktes, welcher letztere Teil dem genannten geprägten Material (31) gegenüberliegt, aufweist.


## Revendications

1. Produit absorbant à transpercement par fluide retardé comprenant une couche absorbante (15) et une couche (13) en matière en feuille gaufrée retardant la pénétration de l'eau qui recouvre une surface principale de la couche absorbante (15), caractérisé en ce que la couche (13) de matière en feuille gaufrée comprend une matière en feuille fibreuse poreuse présentant des zones superficielles extérieures de porosité relativement faible et des zones superficielles intérieures de porosité relativement élevée.

2. Produit absorbant suivant la revendication 1, dans lequel la matière gaufrée (13) est un papier retardant la pénétration de l'eau.

3. Produit absorbant suivant la revendication 2, dans lequel le papier (13) est apprêté avec de l'amidon.

4. Produit absorbant suivant l'une quelconque des revendications 1 à 3, dans lequel la matière gaufrée (13) comprend une série d'ondulations (3) en substance parallèles.

5. Produit absorbant suivant la revendication 4, dans lequel la matière gaufrée (13) comporte de 5 à 30 ondulations (3) par centimètre.

6. Produit absorbant suivant l'une quelconque des revendications précédentes, au moins partiellement enfermé dans une enveloppe perméable au fluide (17).

7. Produit absorbant suivant la revendication 6, dans lequel l'enveloppe (17) est un tissu non tissé.

4

8. Produit absorbant suivant l'une quelconque des revendications précédentes, dans lequel une couche (37) de tissu ouate est incluse dans la couche absorbante (33, 35) entre ses surfaces principales.

9. Produit absorbant suivant l'une quelconque des revendications précédentes, comprenant un revêtement adhésif sur au moins une partie de sa surface externe (41) opposée à la matière gaufrée (31).

FIG. 1.

FIG. 2.

FIG. 3

FIG. 4.

1